# EUROPEAN PATENT SPECIFICATION

(11) **EP 3 057 571 B1**
(45) Date of publication and mention of the grant of the patent: **06.03.2019**
(21) Application number: 13783629.2
(22) Date of filing: 18.10.2013
(51) Int. Cl.: A61K 9/00, A61K 47/06, A61K 47/18, A61K 31/4439

(54) **PROTON PUMP INHIBITOR PASTE COMPOSITIONS**
PROTONENPUMPENHEMMER-PASTENZUSAMMENSETZUNGEN
COMPOSITIONS EN PÂTE CONTENANT UN INHIBITEUR DE LA POMPE À PROTONS

(43) Date of publication of application: 24.08.2016
(73) Proprietor: Norbrook Laboratories Limited, County Down Ireland BT35 6PU (GB)
(72) Inventor: UMRETHIA, Manish, Newry County Down BT35 6PU (GB); HILIAN, Andrew, Newry County Down BT35 6PU (GB)
(74) Representative: HGF Limited
(86) International application number: PCT/GB2013/052730
(87) International publication number: WO 2015/055974

(56) References cited:
- WO-A1-00/50038
- WO-A1-2005/000269
- US-A1- 2002 076 435

## Description

### Technical Field

The present invention relates to novel paste compositions containing a proton pump inhibitor, pharmaceutical formulations comprising said paste compositions and their use for inhibiting gastric acid secretion in mammals.

### Background Art

Proton pump inhibitors (PPI) are potent inhibitors of gastric acid secretion by inhibiting H⁺K⁺ -ATPase, the enzyme involved in the final step of hydrogen ion production in the parietal cells. Several PPI, such as omeprazole, lansoprazole, pantoprazole and leminoprazole, and their processes of making have been described in the art. *See* EP005129, EP174726, EP166287 and GB2163747. A number of pharmaceutical formulations containing PPI have also been disclosed. *See* EP247903.

Some PPI formulations have been proposed for veterinary use. For instance, an oral composition containing a PPI in the form of enteric coated dry particles mixed with a dry gelling agent is described in EP0696921. These enteric formulations are regrettably difficult to administer and expensive to produce. Another example of a PPI formulation is reported in US5708017, which discloses an oral omeprazole paste suitable for veterinary application. However, this paste composition is unstable unless a thickening agent is present.

### Summary of the Invention

The present invention discloses paste compositions comprising a proton pump inhibitor, a basifying agent and a mineral oil-based vehicle useful for the treatment and prevention of diseases and conditions related to gastric acid secretion abnormalities. Surprisingly, the paste compositions thus formulated do not require the addition of a thickening agent, as is the standard practice in the art, in order to be stable. The elimination
of the thickening agent has also the advantage of facilitating the process of making said compositions and reducing their production costs.

Moreover, the present invention shows that the combination of the basifying agent and mineral oil-based vehicle has a synergistic effect that yields more stable PPI compositions. The compositions of the invention are stable after three years of storage, while comparable products have only a maximum shelf life of two years.

An additional benefit of the compositions of the invention is that a lower amount of the basifying agent is required to attain a stability level comparable to other PPI paste compositions known in the art. In circumstances where large concentrations of the basifying agent could be undesirable, this particular feature of the invention minimizes any unwanted effects and improves the overall safety profile of the paste composition.

In a first aspect, the present invention is directed to a stable paste composition comprising:
i) 1% to 45% w/w of a proton pump inhibitor, wherein the proton pump inhibitor is omeprazole,
ii) 0.05% to 0.1% w/w of a basifying agent, wherein the basifying agent is an alkanolamine basifying agent, and
iii) 54.9% to 98.95% w/w of a mineral oil-based vehicle having density of about 0.769 g/mL to about 0.915 g/mL at 15°C as measured by ASTM D-1298,
for use in the treatment or prevention of gastric ulcers in a subject, wherein the paste composition (a) is absent a thickening agent and (b) is administered directly as an oral pharmaceutical formulation to the subject

Advantageously, the paste compositions of the present invention are stable in the absence of a thickening agent.

### Ranges of Components in the Compositions of the Present Invention

In one embodiment, the proton pump inhibitor is about 20% to about 40% w/w. Preferably, the proton pump inhibitor is about 34% to about 39% w/w.

In another embodiment, the mineral oil-based vehicle is about 59% to about 79% w/w. Preferably, the mineral oil-based vehicle is about 60% to about 65% w/w.

In a further embodiment, the proton pump inhibitor, basifying agent and mineral oil-based vehicle comprises 37%, 0.5% and 62.5% w/w of the paste composition, respectively.

Optionally, the paste composition may contain other additives such as flavoring and coloring agents to facilitate its administration, presentation and storage. Other additives do not include thickening agents.

The basifying agent is a pharmaceutically acceptable alkanolamine (e.g. monoethanolamine, diethanolamine, triethanolamine, isopropylamine, diethylamine, trimethylamine). More preferably, the basifying agent is monoethanolamine.

The mineral oil-based vehicle may have a density of about 0.820 g/mL to about 0.895 g/mL at 15°C according to the ASTM D-1298 protocol. For example, the mineral oil-based vehicle may have a density of about 0.840 g/mL to about 0.895 g/mL, and more preferably, of about 0.855 g/mL to about 0.865 g/mL at 15 °C as measured by ASTM D-1298.

The preferred mineral oil-based vehicle for use in the compositions of the present invention is liquid paraffin.

In a further embodiment, the paste compositions of the invention may comprise optionally a coloring (e.g. food yellow no. 5, food red no. 3, food blue no. 2, food lake dye, titanium dioxide, red iron oxide, yellow iron oxide) or flavoring (e.g. caramel, carrot, apple, cinnamon oil) agent. The paste compositions of the invention may also include additional ingredients commonly used in the preparation of human and veterinary products. For example, sweeteners (e.g. sugar, saccharin), preservatives (e.g. parabens), antioxidants (e.g. BHT, BHA) and dispersants (e.g. calcium stearate) can be added to the compositions.

### 1. Definitions

The term "excipient" as used herein, means any component, other than the active substance(s) intentionally added to the formulation of a dosage form. Exemplary excipients are disintegrants, lubricants, plastizicers, binders, fillers, colorants, flavor masking agents, flavoring agents, stabilizers, foaming agents, sweeteners, pore-forming agents, acids (e.g. citric acid, tartaric acid), sodium chloride, a bicarbonate (e.g. sodium, potassium), sugars and alcohols. Some excipients can serve multiple purposes (e.g. filler and disintegrant). *See* US Pharmacopoeia. As used herein, the term "excipient" does not specifically include thickening agents.

The term "liquid paraffin" as used herein, refers to a liquid mixture of C₁₅-C₄₀ alkanes of non-vegetable origin having a density of about 0.855 g/mL to about 0.865 g/mL at 15°C according to the ASTM D-1298 protocol and a kinematic viscosity of about 65 mm²/s to about 75 mm²/s at 40°C according to the ASTM D-445 protocol.

The term "ASTM-XXXX" as used herein, refers to the standard measurement and testing procedures set by ASTM International. In particular, ASTM D-1298 refers to D1298-12b Standard Test Method for Density, Relative Density, or API Gravity of Crude Petroleum and Liquid Petroleum Products by Hydrometer Method [ASTM Volume 05.01, February 2013], and ASTM D-445 refers to D445-12 Standard Test Method for Kinematic Viscosity of Transparent and Opaque Liquids (and Calculation of Dynamic Viscosity) [ASTM Volume 05.01, February 2013].

The terms "prevent", "preventing" and "prevention" as used herein, refer to inhibiting the inception or decreasing the occurrence of a disease in a subject. Prevention may be complete (e.g. the total absence of pathological cells in a subject) or partial. Prevention also refers to a reduced susceptibility to a clinical condition.

The term "subject" as used herein, refers to a mammalian, such as a human being, a non-human primate (e.g. chimpanzees, apes, monkey species), a farm animal (e.g. cattle, sheep, pigs, goats, horses), a domestic mammal (e.g. dogs, cats) or a laboratory animal (e.g. mice, rats, guinea pigs). The term does not denote any particular age or sex.

The term "thickening agent" as used herein, refers to a compound which increases the viscosity of a solution without substantially modifying its other properties. Examples of thickening agents include, but are not limited to, carrageenan, guar gum, gelatine, polyvinylpyrrolidone, hydroxypropylmethylcellulose, hydroxyethylcellulose, hydroxypropylcellulose, traganth, alginate, carboxymethylcellulose calcium or sodium, silicon dioxide, castor oil and its derivatives (e.g. hydrogenated castor oil), and aluminum hydroxide.

The term "treat" or "treatment" as used herein, refers to the administration of a composition or formulation of the invention to a subject in order to control the progression of a disease after its clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state, and remission (both partial and total). The control of progression of the disease also involves an extension of survival, compared with the expected survival if treatment is not applied.

The term "therapeutically effective amount" as used herein, refers to any amount of a compound, composition or formulation which, when administered to a subject: i) prevents the inception or recurrence or ii) causes the reduction or remission of the disease or condition against which the compound, composition or formulation is effective.

### 2. Proton pump inhibitors

The PPI is omeprazole, a pharmaceutically acceptable salt, isomer, tautomer, enantiomer or a combination thereof.

In general, the PPI of the invention are acidic in nature and may form base salts with various pharmacologically acceptable cations such as alkali metal or alkaline earth metal salts. Examples of basic salts include, but are not limited to, the calcium, magnesium, sodium and potassium salts of the compounds of the present invention.

The PPI of the invention shall be interpreted to include also certain of their forms or variations. Said compounds may have asymmetric centers and, therefore, exist in different enantiomeric or diastereomeric forms. Thus, any given compound referred to herein is intended to represent any one of a racemate, one or more enantiomeric forms, one or more diastereomeric forms or a combination thereof. Likewise, stereoisomerism or geometric isomerism about the double bond is also possible, therefore in some cases the molecule could exist as *cis* or *trans* isomers. If the molecule contains several double bonds, each double bond will have its own stereoisomerism, that could be the same as, or different to, the stereoisomerism of the other double bonds of the molecule. Furthermore, the PPI of the invention may exist as atropisomers. All the stereoisomers of the PPI of the invention, including their enantiomers, diastereoisomers, geometric isomers, atropisomers and the combinations thereof, are considered within the scope of the present invention.

Furthermore, the PPI of the invention may exist as tautomers. Specifically, the term tautomer refers to one of two or more structural isomers of a compound that exist in equilibrium and are readily converted from one isomeric form to another. Common tautomeric pairs include, but are not limited to, amine-imine, amide-imidic acid, keto-enol, and lactam-lactim.

### 1. Process of making paste composition

Paste compositions for use in the invention, can be prepared by mixing a PPI, a basifying agent and a mineral oil-based vehicle.

### 2. Pharmaceutical formulations

The present invention provides a pharmaceutical formulation comprising the paste composition of the invention and at least one excipient for use in the treatment of gastric ulcers. Preferably, the pharmaceutical formulation is suitable for oral administration such as an oral solution, oral suspension, feed premix, gels, capsule or bolus. The formulations of the invention may be produced following methods known in the art. *See* Gunnar A, Ed., "Remington: The Science and Practice of Pharmacy" 20th ed. (Lippincott Williams & Wilkins, Philadelphia, PA, US, 2003).

Other suitable excipients for the stabilization of PPI include, in particular, buffers with a pH value of 6 or higher, preferably between pH 8 and 11, such as buffer mixtures comprising sodium phosphate, sodium hydrogen phosphate, sodium dihydrogen phosphate, sodium carbonate, sodium hydrogen carbonate and sodium hydroxide or a combination thereof.

### 5. Use of paste composition and formulation for the treatment or prevention of diseases or conditions

In another aspect, the present invention refers to the use of a paste composition according to the invention or a pharmaceutical formulation containing said paste for inhibiting gastric acid secretion, including the treatment or prevention of diseases or conditions due to an increased gastric acid secretion. In a particular embodiment, the disease or condition is gastroesophageal reflux disease, gastric and duodenum ulceration or gastritis. Alternatively, the present invention refers to a method of inhibiting gastric secretion in a subject which comprises administering to the subject a therapeutically effective dose of a paste composition or pharmaceutical formulation according to the invention. Preferably, the subject is a human being or a horse. More preferably, the subject is a horse.

Preferably, the paste composition or formulation of the invention is administered periodically, such as on a daily basis. However, a single treatment or administration of the paste composition or formulation over one or two days (e.g. once daily for one or two days), such as prior to or during a stressful event, is also contemplated.

The effective amount of the PPI administered should be about 0.1 to about 8 mg per kg body weight. Preferably, the PPI is administered in relatively low doses. In addition, the paste may be administered in the form of a formulation for controlled release and long lasting delivery, in which case the administration can be less frequent (e.g. weekly, monthly).

The dosage regimen may vary with time depending on the therapeutic objective sought. For instance, for the treatment of gastric ulcers, a relatively high dose of 4 to 5 mg PPI per kg body weight per day may be used at the beginning of treatment followed by a lower dosage regimen of 1 mg PPI per kg body weight per day after the subject has been stabilized. On the other hand, a basal dosage regime of 1 mg PPI per kg body weight per day may suffice to prevent the occurrence or recurrence of gastric ulcers.

The duration of the administration for preventing ulcers may last several days. Preferably, the administration is continuous, either for life or at least during the whole period where the subject is or is suspected to be under stress conditions or other conditions which may increase the risk of formation of ulcers.

Preferably, the paste composition or formulation is administered to a subject having a substantial risk of developing gastrointestinal ulcers. For instance, the administration to horses is preferably conducted during periods of stress, training, transportation, change in environment (e.g. weather changes, housing changes) or pregnancy.

According to the invention, the paste composition can be administered under any suitable formulation for delivery, preferably, for oral delivery. Preferably, the paste composition is administered directly as an oral pharmaceutical formulation.

This invention is further illustrated by the following examples which should not be construed as limiting.

### Examples 1, 2 and 3 Stable omeprazole paste compositions

First, yellow iron oxide was added to liquid paraffin and mixed slowly for 15 minutes or until a uniform dispersion was obtained. Then, monoethanolamine and cinnamon leaf oil were added to the blend and mixed slowly for 10 minutes until uniformly dispersed. Omeprazole was incorporated to the blend in small amounts and mixed at low speed until a smooth and uniform paste was obtained. Compositions containing 37%, 38% and 39% w/w omeprazole were prepared adjusting the liquid paraffin content accordingly. *See* Table 2. All compositions were stable after 36 months at 25°C and 60% relative humidity.

**Table 2**

| Ingredients | Example 1 (w/w) | Example 2 (w/w) | Example 3 (w/w) |
|---|---|---|---|
| Omeprazole | 37% | 38% | 39% |
| Monoethanolamine | 0.05% | 0.05% | 0.05% |
| Yellow iron oxide | 0.02 % | 0.02 % | 0.02 % |
| Cinnamon leaf oil | 0.1% | 0.1% | 0.1% |
| Liquid paraffin | 62.65% | 61.65% | 60.65% |

### Example 4 Comparative analysis of paste compositions

Paste compositions containing omeprazole, several oily liquid vehicles and monoethanolamine were prepared according to the protocol above. The compositions were stored at 25°C and 60% relative humidity. Their stabilities were assayed after 3 months. *See* Table 3.

**Table 3**

| | Assays | | | | | |
|---|---|---|---|---|---|---|
| Ingredients | 1 (w/w) | 2 (w/w) | 3 (w/w) | 4 (w/w) | 5 (w/w) | 6 (w/w) |
| Omeprazole | 37% | 37% | 37% | 37% | 37% | 37% |
| Monoethanolamine | 0.1% | 0.02% | 0.05% | 0.1% | 0.05% | 0.05% |
| Polyethylene glycol | 62.9% | 42.98 % | ----- | ----- | ----- | ----- |
| Neobee oil | ----- | ----- | 62.95 % | ----- | ----- | ----- |
| Castor oil, hydrogenated | ----- | 20% | ----- | 1.25% | ----- | ----- |
| Soybean oil | ----- | ----- | ----- | 61.65% | 62.95 % | ----- |
| Liquid paraffin | ----- | ----- | ----- | ----- | ----- | 62.95% |
| | | | | | | |
| Condition | Unstable | Unstable | Unstable | Stable | Unstable | Stable |

The data above shows that, in the absence of a thickening agent, PPI paste compositions based on vegetable oils or polymeric glycol vehicles are unstable, *vide supra* assays 1-3, and 5 versus assay 4. However, when a mineral oil vehicle is employed instead, the resulting paste composition is stable without requiring the addition of any thickening agent, *vide supra* assay 6. This last approach also produces a paste composition with a longer shelf life.

Moreover, the use of a mineral oil vehicle to prepare PPI paste compositions makes possible reducing the weight content of any basifying agents present in the mixture. For instance, a minimum content of 0.1% w/w of the basifying agent monoethanolamine is required to maintain the stability of an omeprazole paste composition when a vegetable oil or polymer vehicle is used. However, when liquid paraffin is incorporated to the paste, the monoethanolamine content may be reduced 50% and still yield a stable omeprazole composition.

## Claims

1. A stable paste composition comprising:
i) 1% to 45% w/w of omeprazole,
ii) 0.05% to 0.1% w/w of an alkanolamine basifying agent, and
iii) 54.9% to 98.95% w/w of a mineral oil-based vehicle having a density of about 0.769 g/mL to about 0.915 g/mL at 15°C as measured by ASTM D-1298,
for use in the treatment or prevention of gastric ulcers in a subject, wherein the paste composition (a) is absent a thickening agent and (b) is administered directly as an oral pharmaceutical formulation to the subject.

2. The composition according to claim 1, wherein the alkanolamine basifying agent is monoethanolamine.

3. The composition according to claim 1, wherein the mineral oil-based vehicle is liquid paraffin.

## Patentansprüche

1. Stabile Pastenzusammensetzung, umfassend:
i) 1 bis 45 Gew.-% Omeprazol,
ii) 0,05 bis 0,1 Gew.-% eines Alkanolamin-Alkalisierungsmittels, und
iii) 54,9 bis 98,95 Gew.-% eines Vehikels auf Mineralölbasis mit einer Dichte von etwa 0,769 g/ml bis etwa 0,915 g/ml bei 15 °C, gemessen gemäß ASTM D-1298,
zur Verwendung bei der Behandlung oder Prävention von Magengeschwüren in einem Subjekt, wobei die Pastenzusammensetzung (a) kein Verdickungsmittel enthält und (b) direkt als eine orale pharmazeutische Formulierung an das Subjekt verabreicht wird.

2. Zusammensetzung nach Anspruch 1, wobei es sich bei dem Alkanolamin-Alkalisierungsmittel um Monoethanolamin handelt.

3. Zusammensetzung nach Anspruch 1, wobei es sich bei dem Vehikel auf Mineralölbasis um flüssiges Paraffin handelt.

## Revendications

1. Composition de pâte stable comprenant :
i) 1 % à 45 % p/p d'oméprazole,
ii) 0,05 % à 0,1 % p/p d'un agent d'alcalinisation alcanolamine,
iii) 54,9 % à 98,95 % p/p d'un véhicule à base d'huile minérale ayant une densité d'environ 0,769 g/mL à environ 0,915 g/mL à 15 °C telle que mesurée selon ASTM D-1298, destinée à être utilisée dans le traitement ou la prévention d'ulcères gastriques chez un sujet, dans laquelle la composition de pâte (a) est dépourvue d'un agent épaississant et (b) est administrée directement comme une forme pharmaceutique orale au sujet.

2. Composition selon la revendication 1, dans laquelle l'agent d'alcalinisation alcanolamine est la monoéthanolamine.

3. Composition selon la revendication 1, dans laquelle le véhicule à base d'huile minérale est la paraffine liquide.
